(19) Europäisches Patentamt
European Patent Office
Office européen des brevets

(11) Publication number: **0 226 288**
**A2**

(12)

# EUROPEAN PATENT APPLICATION

(21) Application number: 86307747.5

(22) Date of filing: 08.10.86

(51) Int.Cl.4: C 12 Q 1/68
C 12 N 15/00, C 12 N 7/00
C 12 P 19/34

(30) Priority: 09.10.85 US 785798
29.05.86 US 868351

(43) Date of publication of application:
24.06.87 Bulletin 87/26

(84) Designated Contracting States:
AT BE CH DE ES FR GB GR IT LI LU NL SE

(71) Applicant: COLLABORATIVE RESEARCH INC.
Two Oak Park
Bedford Massachusetts 01730(US)

(72) Inventor: Barker, David F.
45 Kilgore Avenue Medford
Massachusetts 02155(US)

(72) Inventor: Braman, Jeffrey C.
Alberta Drive Hudson
Massachusetts 01749(US)

(72) Inventor: Buchwald, Manuel
353 Riverdale Avenue Toronto
Ontario MFJ184(CA)

(72) Inventor: Keller, Helen-Donis
80 Park Street Brookline
Massachusetts 02146(US)

(72) Inventor: Knowlton, Robert G.
30 Shirley Street Lexington
Massachusetts 02173(US)

(72) Inventor: Schumm, James W.
27 Moningside Avenue Natick
Massachusetts 0760(US)

(72) Inventor: Tsui, Lap-Chee
170,Elisabeth Street,Hospital Children
Torronto/Ontario M5G 1X8(CA)

(74) Representative: Shipley, Warwick Grenville
Michael et al,
VENNER, SHIPLEY & CO. 368 City Road
London EC1V 2QA(GB)

(54) **Means and method of testing for cystic fibrosis based on genetic linkage.**

(57) The present invention provides a method of determining presence or absence of one or more cystic fibrosis alleles in a DNA sample taken from an individual having a family genetic background in which family, DNA of ancestors in the family line contains identified cystic fibrosis causing allele genes, said method comprising,

restriction digesting a DNA sample to be tested, which DNA sample is from an individual having a family genetic background in which family ancestors of the family have DNA which contains identified cystic fibrosis allele;

apply a probe to said DNA sample which probe hybridizes within less than 50 centimorgans of a cystic fibrosis gene in said family DNA,

and determining whether or not hybridization has taken place in a manner consistent with the presence of one or more cystic fibrosis alleles in said sample.

EP 0 226 288 A2

Croydon Printing Company Ltd.

# MEANS AND METHOD OF TESTING FOR CYSTIC FIBROSIS BASED ON GENETIC LINKAGE

Cystic Fibrosis (CF) is the most common lethal genetic disease present in United States populations with a frequency of about 1 in 2000 live births in the United States. The allele frequency in the population is almost 1 in 20 individuals. It is classified lethal since clinical features of the ailment are often sufficiently serious to limit life expectancy to the second or third decade of life. The course of the disease can be quite variable among patients but early diagnosis and improved therapies have led to an increase in life expectancy.

Cystic Fibrosis is a genetic disease that is transmitted from parents to offspring as an autosomal recessive trait. An autosomal recessive trait is one in which each parent contributes a mutant or defective allele to the child who then expresses the disorder. The parents of a Cystic Fibrosis offspring can be afflicted wth Cystic Fibrosis or more usually are carriers of the gene defect but do not show any clinical symptoms of the disease. Due to the lack of any diagnostic test, the parents are unaware of their CF carrier status until a CF child is produced.

Although Cystic Fibrosis is generally acknowledged as a single gene disorder transmitted as an autosomal recessive, significant differences in the severity of the disorder among individuals suggest either genetic heterogeneity (one parent alone can transmit a dominant CF gene) or that some mutant alleles are more debilitating than others. Alternatively, other researchers argue that several different loci (genes) could produce the phenotype (disease) classified as CF.

Clinically, CF is a generalized disease of exocrine glands that can cause body problems having symptoms which include pancreatic insufficiency, pulmonary obstruction, respiratory tract infection and other severe disorders.

Diagnosis of CF is most commonly limited to individuals who clearly display symptoms of the disease. An initial indication of CF is usually suspected from characteristic pulmonary and gastrointestinal findings, or a positive family history. The current accepted confirmation of diagnosis of CF is documentation of an elevated sweat electrolyte concentration. The only acceptable sweat test method is the quantitative pilocarpine iontophoresis sweat test. This test has not been useful as a general screen because of the high cost and difficulty in obtaining an adequate quantity of sweat from newborns.

A number of homozygote CF afflicted persons and CF carrier (heterozygote) screening tests other than the sweat test have been proposed over the past few years but none have been instituted in large scale screening services. The most widely used postnatal screening tests are those based on the presence of increased amounts of albumin or protein in meconium (mucous like secretions found in the intestines) and which reflect a reduction or absence of pancreatic proteolytic enzymes. Unfortunately, these tests often do not detect the 15-20 percent of homozygotes with normal pancreatic function. Additional problems include occasional false negative values in persons with pancreatic insufficiency and false positive values in the presence of prematurity of melena. The simultaneous measurement of beta-D-fucosidase and lactase in meconium may increase the specificity of the test. A recently proposed screening method uses dried blood spot assay for immunoreactive trypsin. However, an extensive evaluation project including screening of over 90,000 newborns found the predictive value of this latter test to be only 17% (Hammond et al., 1984).

Other tests currently being carried out including bioassays for sodium transport inhibitory factors and mucociliary factors have been difficult to standardize and are not useful for heterozygote screening. Further, studies that initially showed increased steroid and ouabain resistance or abnormal sodium transport in fibroblasts from CF homozygotes and heterozygotes were later shown to be not reproducible. A prenatal diagnosis testing scheme utilizing monoclonal antibodies to three isoenzymes of alkaline phosphatase from amniotic fluid of second trimester pregnancies has been proposed. Low enzyme levels are scored as the CF phenotype. The false positive values were listed as 6% and 23/26 CF cases were found to have 1/2 median value for the corresponding week of gestation (Brock et al., 1985). This proposed test awaits large scale evaluation and has been questioned for its lack of precision.

Recently a serum protein activity (paraoxonase or PON) has been shown to be genetically linked to cystic fibrosis by studies of coinheritance of the marker in families segregating CF (Eiberg, H., Schmiegelow, K., Tsui, L.-C., Neiburhr, E., Phelan,

P.D., Williamson, R., Warwick, W., Schwartz, M., Koch, C. and Mohr, J. Cytogenet. Cell Genet., in press, and Schmeigelow, K., Eiberg, H., Buchwald, M., Tsui, L.-C., Phelan, P.D., Williamson, R., Warwick, W., Neiburhr, E., Mohr, J., Schwartz, M. and Koch, C. Lancet, in press). However, it is unlikely that this enzyme activity will furnish a diagnostic test because prenatal samples cannot be analyzed. While many biochemical parameters hve been analyzed, no generally accepted reproducible prenatal test is available for homozygotes nor are there any generally accepted heterozygote screening tests available.

In additional to the lack of reliable postnatal homozygote diagnostic tests, there are no reliable prenatal homozygote tests currently available and no satisfactory heterozygote analysis (carrier testing) can be done at the present time. Diagnosis in advance of symptoms would clearly be of benefit for treatment and as a means of providing information for genetic counseling.

Because of the lack of availability of heterozygote testing and prenatal diagnosis, probabilities based on mendelian genetics are the only information that can be reliably given to families with a positive family history of CF. For example, parents who have given birth to a CF child are obligate heterozygotes with one out of four offspring inheriting both mutant alleles and developing CF. With the latter information, parents will only know that with each new birth the risk factor is 25% for CF and 50% for carrier status. Diagnostic tests based on mendelian genetics are not able to give a parent high probability whether the at risk individual is a carrier, has the disease or is free of the disease gene.

Recently gene linkage testing for a dominant genetic trait, i.e. Huntington's Chorea has been carried out by others using the DNA probes in hybridization test steps. However, there has been no accepted linkage test used for determining presence or absence of autosomal recessive traits such as disease and specifically cystic fibrosis disease.

It is an object of the present invention to provide a method for detecting autosomal recessive trait genes.

It is a further object of the present invention to provide a detection method that utilizes genetic linkage.

Another object of the present invention is to provide a cystic fibrosis disease diagnostic test which could determine whether an individual is a carrier, has the disease or is free of the disease gene.

It is an additional object of the present invention to provide a means and method for identifying whether or not a cystic fibrosis gene is present in a DNA sample.

Another object of the present invention is to provide a method for linkage to the cystic fibrosis gene using restriction fragment length polymorphism (RFLP) probes.

According to the invention, a DNA probe is provided which has a PIC value of at least 0.58. The probe hybridizes within less than 50 centimorgans of a cystic fibrosis gene locus and preferably within 10 centimorgans of the locus. The probe can be Lam4-917.

In a method of identifying whether or not an autosomal recessive trait gene is present in a DNA sample taken from an individual, a probe is applied under hybridizing conditions to a DNA sample which probe identifies an autosomal recessive trait gene locus specific to an autosomal recessive trait gene in the DNA if present. It is then determined whether or not the probe has hybridized to the DNA. Preferably the gene is a gene which causes disease in humans as for example a cystic fibrosis gene. The hybridization is examined to determine if it has taken place in a manner consistent with the presence of one or more cystic fibrosis alleles. This is done by comparing results of hybridization with the results obtained from DNA of the parents of the individual from whom the sample to be tested was taken, so that hybridization at similar positions on a solid support with respect to each parent, indicates the presence or absence of a cystic fibrosis allele obtained from each parent in the DNA tested.

Preferably a method of determining the presence or absence of one or more cystic fibrosis alleles in a DNA sample taken from an individual having a family genetic background in which family, DNA of ancestors in the family line contain cystic fibrosis genes, comprises obtaining a DNA sample from the individual, restriction digesting the DNA sample to be tested and applying a probe to the DNA sample which probe hybridizes within less than 50 centimorgans of a cystic fibrosis gene in the family DNA. There is then a determination made as to whether or not hybridization has taken place in a manner consistent with the presence of one or more cystic fibrosis alleles in the sample.

It is a feature of this invention that a test can be carried out on a pre-born fetus, an individual showing no symptoms of the autosomal recessive trait or any individual so long as there is a family of ancestor's DNA available for comparison hybridization with a selected DNA probe which hybridizes with an autosomal recessive trait gene in that family. In most instances diagnostic tests have a high degree of accuracy when positive and a higher degree of accuracy when negative than hithertofore used diagnostic procedures The testing can be carried out with minimum inconvenience to the parties involved and with small samples of DNA taken from a variety of body locations.

## BRIEF DESCRIPTION OF THE TABLES

Table 1 contains the locus revealed by probe Lam4-9.7.

Table 2 contains the restriction maps of Lam4-917 and of the genomic DNA segment of the locus. The 17.5 kilobase (kb) cloned human segment is represented by thick lines in both the Lam4-917 phage and the genomic representations. The letters B, E, and H indicate the sites for digestion with restriction endonucleases BamHI, EcoRI, and HindIII, respectively. Numbers below the phage diagram indicate the distance, in kb, from the left end of Lam4-917; numbers in the genomic diagram indicate the size, in kb, of fragments generated by HindIII digestion and detected by Southern gel transfer hybridization analyses. The parentheses designate the polymorphic HindIII site revealed by probe Lam4-917.

Table 3 contains the linkage relationships of probe Lam4-917 to CF and probe Lam4-917 to PON. The maximum likelihood estimate is obtained for Θ at 0.14 with a lod (z) score of 3.96 (odds ratio: 9,100:1), if recombination frequencies are considered equal in male and female. The confidence interval for Θ is between 0.07 and 0.25 (19). The recombination fraction is close to the frequency of recombination detected by counts of the number of apparent recombinant chromosomes divided by the number of informative chromosomes among CF children (13/101).

Table 4 contains a family pedigree in a typical counseling situation. The darkened areas (1 and 4) represent CF alleles and the light areas (2 and 3) normal alleles.

Table 5 contains a family pedigree following diagnostic analysis using probe Lam4-917. The darkened areas represent CF alleles and the light areas normal alleles A, B, C, D are alleles of Lam4-917 generated by HindIII digestion and E, F, G, H are alleles of Lam4-917 generated by HincII digestion.

Table 6 contains a 2-point linkage map of chromosome 7 based on mapping with CEPH (Centre E'etude du Polymorphisms Humain) families where θ is recombination frequency between two markers and the LOD is as described below. Certain of the probes shown in this Table are further described in Table 7.

Table 7 contains a listing of flanking markers within 10 centimorgans of the CF gene. The Table contains the alleles revealed for each marker and their fragment links.

## BRIEF DESCRIPTION OF PREFERRED EMBODIMENTS

The principle of genetic linkage is used to detect and follow autosomal recessive disease causing genes. The test does not require any knowledge of the biochemical basis of the disorder in order to test for its presence in an at risk individual or to identify a carrier of a mutant allele. The genetic location of such a disease gene is first determined by linkage studies with DNA RFLP probes and the probes that are linked are then used to study the inheritance of the disease gene in families known to be segregating the disease.

In cases where a single gene causes a disorder only one chromosomal location needs to be followed with genetically linked RFLP probes. In cases where more than one gene can give rise to the defect or phenotype, several chromosomal loci will necessarily be monitored unless it is possible to distinguish which gene is segregating in the particular family under study. The gene which is segregating may be deduced from the phenotype of the affected child in the family.

This invention defines the location of a cystic fibrosis gene by linkage studies using a RFLP DNA probe and a method of diagnosis for CF using DNA hybridisation in gel transfer methods. It appears that the chromosomal location, i.e. CF locus, is responsible for most if not all of the CF cases included in the study herein. In addition, this test utilizes a simple technology and need only be performed once in the lifetime of an individual (who is at risk, putative carrier or normal). The information determined may have value for any future members of the family who may be at risk for cystic fibrosis.

Phenotypically neutral DNA sequence variations occur among individuals of any given species and arise from the insertion or deletion of DNA segments or, more commonly, by single nucleotide base changes. Some sequence differences change restriction endonuclease recognition sites or their spacing. The sequence difference is then revealed as variation in the length of restriction fragments recognized by a cloned DNA probe when the DNA of two or more individuals is examined. Preferably total genomic DNA is cleaved with a restriction enzyme, separated according to size by electrophoresis, transferred and bound to a solid support such as a nylon membrane and hybridized to a radioactively labeled cloned DNA probe. The restriction fragment length polymorphism (RFLP) is visualized by autoradiography as a difference in the pattern of bands produced by hybridization of the probe to genomic DNA.

Family studies of ancestors of individuals at risk have shown that the variations in fragment lengths, RFLPs, are inherited in a Mandelian fashion with one form, allele coming from each parent. Since the RFLPs are stably inherited they can be used as genetic markers useful in the construction of a human genetic linkage map as proposed by Botstein, D., White, R.L., Skolnick, M. and Davis, R.W. (1980) AM. J. Hum. Genet. 32, 314-331. The markers can be identified and located with RFLP probes which hybridize with the RFLP markers to reveal polymorphism at a single locus (e.g. a particular segment on a given chromosome). These same genetic markers can also be used to locate and follow the inheritance of certain traits including diseases such as cystic fibrosis. The closer the RFLP marker location is to the disease gene of interest the more likely it is to be co-inherited with the disease gene. For example, if a RFLP locus is at a genetic distance of 10 centimorgans from a gene (recombination fraction of 10% or physical distance of approximately 10 million nucleotides), 90% of the time an RFLP allele from this locus will be coinherited with the disease locus since they are on the same segment of DNA but 10% of the time a crossover event will occur during meiosis such that a different RFLP allele from the locus will become attached to the DNA segment carrying the disease gene. At a genetic distance of 50 centimorgans from a gene, markers are said to be unlinked since a recombination event will occur approximately 50% of the time for each meiosis. In addition, it is very important that a large number of alleles at similar frequencies be present at a locus so that in any random mating there is a high probability that each parental chromosome contribution can be followed in the offspring. This measure of heterozygosity is referred to as the polymorphism information content or PIC (Botstein, et al. (1980) supra).

The present test utilizes cloned DNA probes that reveal polymorphism at a locus that is genetically linked to a cystic fibrosis locus. Therefore the inheritance of the disease cystic fibrosis can be detected by analysis of the RFLP alleles known to be cosegregating in families. Since the particular RFLP allele will vary from family to family the particular allele coinherited in an affected individual is the basis of the diagnosis in the family. Once this allele is identified it can be followed in all other individuals at risk that are descendants of the same two parents in the family. Thus the test is a linkage test that is preferably used as a family study rather than a population screening test.

In any linkage test there is the possibility that the two chromosomes that are being passed on to the child from the parents cross over (e.g. the mother's chromosomes break and rejoin with each other, exchanging genes). If there is only 1 marker in the test then the probability of a wrong diagnosis is simply related to the distance between the marker and the disease gene. Typically such a number might be 5% per parental chromosome resulting in a 10% error rate (i.e if the marker is 5 centimorgans (5% recombination) from the disease gene and since CF is a recessive, then, two disease genes are being passed on per generation). That is, 10% of the time a crossover will occur and it can't be determined that it has occurred, hence the 10% error rate.

With flanking markers one can tell when a crossover has occurred in the region. For example, if one marker is 5 centimorgans from the disease gene and a marker on the other side of the cystic fibrosis gene is 3 centimorgans from the disease gene then, 16% of the time, a crossover event will occur in the transmission of the parental chromosomes to the child and the crossover will be detected (8% recombination for each parental chromosome or 16% total). The important difference here is that the accuracy of the flanking marker test is 99.7% versus 90% for the single marker test. The reason it is not 100% is due to cases in which two simultaneous crossovers between each of the markers and the disease gene (also known as a double crossover) that could occur, and this is calculated to be 0.3% of cases. So in our example 84% of the population (100% - 16% recombinants) will get a diagnosis that is 99.7% accurate (i.e. 0.3% error rate).

What can be done for the 16% of the population that has a documented crossover in an offspring at risk? These people can be given a partial diagnosis (in addition to knowing that a crossover has occurred) and in some cases this will help make a better risk assessment. One can note that there is a 5/8 probability that the crossover occurred in the interval between the marker, that is, 5 centimorgans from the disease gene and a 3/8 probability that the crossover occurred in the interval between the marker 3 centimorgans from the disease gene.

BAD ORIGINAL

Therefore, there is a probability of 38% that the child has CF and a 62% chance that it is a carrier. Depending on the marker distances from the disease gene these percentages will be of more or less usefulness in making a risk assessment. Of course the above illustration assumes that the markers are fully informative, that is, each parental chromosome can be distinguished at both marker locations.

The RFLP alleles are preferably detected by DNA hybridization with cloned preferably radioactively labeled DNA probes in gel transfer analysis of genomic DNA from family members, e.g. both parents, an affected child and the individual at risk.

The basis for this system of presymptomatic diagnosis for CF is the determination that a particular single-copy DNA probe called Lam4-917 defines a restriction fragment length polymorphism when used in gel-transfer hybridization methods and that the locus defined by this RFLP is linked to a gene that is responsible for a large fraction, if not all, inherited CF in humans and among caucasians. The linkage test can determine, to the precision indicated below, whether a child (even *in utero*) has inherited the defective (i.e. disease-causing) allele from one or both of its parents. If it inherited the disease allele only from one parent, it will be a "carrier" (not diseased but capable of passing on the disease to its offspring); if it inherited disease alleles from

both parents, it will have the disease; if from neither parent, then it will be free of CF.

The usefulness of the diagnostic test based on linkage is determined by three factors:

1. the degree of polymorphism as measured by the PIC;
2. the degree of linkage of the marker to the disease locus, measured by the recombination fraction observed between the RFLP locus and the disease gene, or, more conventionally by the genetic distance in centimorgans between them; or
3. the fraction of the disease caused by the particular disease gene: e.g. CF is caused by a single gene or, alternatively, some fraction of families with inherited CF may suffer because of another CF gene.

The more polymorphic an RFLP locus is, the more likely that a random individual will harbor different RFLP alleles than his or her spouse. In order for linkage to be followed, the RFLP probe which carries a radioactive or other marker capable of being detected, must be informative (i.e. heterozygous), and thus the PIC measures the usefulness of the probe in diagnosis. The probe Lam4-917 has a PIC of 0.58 (see Table 1), meaning that in its present form the probe will be useful in more than 1/2 of all the families to which it is applied. The PIC of a particular probe can be improved by techniques familiar to one skilled in the art, and thus the present invention preferably utilizes Lam4 917 but improvements in its PIC or other probes and their improved PICs may be used.

Probes having PIC values of lower than 0.58 can be useful. However, when PIC values are below 0.58 it is preferred to use combinations of two or more probes in diagnosis in order to improve accuracy.

The degree of linkage (i.e. recombination fraction) between the locus defined by 917 and the major and possibly only CF locus is estimated to be about 0.15, meaning that 15% of the time the 917 allele in coupling with the CF allele will be exchanged so that the alternative allele of 917 is transmitted instead. Thus the linkage test using Lam4-917 in its present form can improve the odds of correct genotype to 0.85 x 0.85 = 0.7225 for determination that a sibling of a CF child also has CF; the probability that a diagnosis of complete absence of the gene is in error is 0.15 x 0.15 = 0.0225, or 2.2. This is a distinct improvement over the current state of the art, which predicts odds of 0.25 (i.e. random) that a child has CF and 0.75 that it does not. Since the diseased gene has been located with the linked RFLP 917, the linkage can be improved by techniques familiar to one skilled in the art and other RFLP markers linked to the major CF gene as well as those markers located on the other side of the CF gene can be found using the procedure described for 917. Techniques familiar to one skilled in the art to improve the linkage include "walking" along the chromosome using probe Lam4 917 screening libraries prepared from the chromosome on which the CF locus is located and preparing subsets of genomic libraries by using probe Lam4-917.

Linkage mapping efforts as those described above in relation to Lam4-917, have resulted in the linkage map of chromosome 7 shown in Table 6. Four of the markers are found to be within about 5 centimorgans of CF (designated L4-1033, S002, S158 and S094). These markers are very close to CF and to one another such that recombinants which might be useful for ordering markers with respect to each other and CF are rare making the precise ordering of the markers and their genetic distances from CF subject to some question. The map can be further resolved by such means as developing multilocus linkage analysis techniques and by analysing more family segregation data. Flanking markers as determined thus far and as shown in Table 6 include on one side of the CF locus :

| | | |
|---|---|---|
| S-140 | S-148 | S-241 |
| S-199 | S-201 | S-003 |
| S-134 | L4-544 | |

and on the other side:

S-162, S-167, S-014, S-023, S-019, S-056, S-025, L4-917, S-146, L4-887, S-102, S-065, S-016, S-139, L4-751, S-155, L4-1020, S-060, S-244, S-166, S-161, S-194, L4-966, L4-281

Those markers listed above which are within 50 centimorgans of the CF locus are particularly desirable for use in the methods of this invention.

The above noted markers are all obtained from a chromosome 7 library obtained and treated as described in Example 1. The probes obtained are then analyzed and determined to have the linkage as noted in Table 6.

As used herein, the following terms have their known meaning in the art defined below:

CHROMOSOMAL LOCUS. A position on a chromosome defined either by means which allow physical localization or by linkage to other positions on the same chromosome.

HETEROZYGOTE. An individual possessing two forms, or alleles, of a polymorphic locus. One form is present on each chromosome of a homologous pair.

LETHAL GENETIC DISEASE. An inherited disorder which can lead to the death of the affected individual.

LINKAGE. A relationship between two chromosomal loci such that they are inherited together more than half the time. Linkage of two loci implies that they are located on the same chromosome.

MENDELIAN GENETICS. The study of traits determined by unique genes or sites. Named after Gregor Mendel (1822-84).

OBLIGATE HETEROZYGOTE. An individual who can be determined to be a heterozygote at particular chromosomal loci based on knowledge of the genetic constitution of other individuals in his/her family.

PHENOTYPE. The entire expressed physical, biochemical, and physiological constitution of an individual. When phenotype is used in the context of a single chromosomal locus, it refers to that portion of an individual's constitution which is affected by that locus.

PHENOTYPE. The entire expressed physical, biochemical, and physiological constitution of an individual. When phenotype is used in the context of a single chromosomal locus, it refers to that portion of an individual's constitution which is affected by that locus.

PHENOTYPICALLY NEUTRAL. Having no effect on phenotype.

POLYMORPHISM (or genetic heterogeneity). Alternate forms observed at a chromosomal locus.

SEQUENCE POLYMORPHISM. A polymorphism resulting from differences in the DNA or protein sequence observed in different individuals at the same chromosomal locus.

It has been estimated that approximately 70% of the human genome is comprised of unique i.e. single-copy DNA sequence and 20% mid-repeat sequences (approximate repeat 2-1000 times per genome) while about 10% is highly repeated 50-$10^5$ fold repeat sequences (Strayer et. al., 1985). While polymorphism no doubt exists in all three classes of human sequence, variation between individuals at single-copy sequence regions is most useful for the genetic testing described above. Only unique sequence regions are tested for RFLPs by hybridization of cloned single-copy DNA sequences to restriction endonuclease cleaved DNAs from unrelated individuals in gel-transfer experiments (Wyman, A. and White, R. (1980) Proc. Natl. Acad. Sci. USA 77, 6754-6758; and White et al. 1985).

The probes are preferably derived from whole or partial human genomic libraries which have been constructed and contain cloned DNA segments 10-20 kilobases in length (Lawn, R. W., Fritsch, E. F., Parker, R. C., Blake, G. and Maniatis, T. (1978) Cell 15, 1 157-1174). Putative single copy clones are identified preferably from plaque filter hybridization experiments in which total genomic human DNA is labeled with $^{32}P$ via a nick translation reaction (Rigby, P. W. J., Dieckmann, M., Rhodes, C. and Berg, P. (1977) J. Mol. Biol. 113, 237-254.) and hybridized to filter replicas of bacteriophage clones containing human DNA inserts plated on a lawn of E. coli (Wyman et al. (1980) supra). Bacteriophage plaques that show hybridization signals contain middle repetitive to highly repetitive human DNA sequences; these are avoided in favor of those that contain human DNA but do not hybridize to the total human DNA probe.

Each candidate single copy human DNA clone can be tested for the ability to reveal polymorphism by hybridization to restriction enzyme cleaved genomic DNA from random unrelated individuals. The bacteriophge clones containing the human DNA inserts are radioactively labeled with $^{32}P$ via nick translation and hybridized in gel transfer experiments to genomic DNAs. A standard screening blot consisting of 5 unrelated individuals cleaved with each of 6 restriction enzymes (e.g. MspI, TaqI, HindIII, EcoRI, BglII, BamHI) is efficient in detecting polymorphism. Since 10 chromosomes are scored, a two allele polymorphism (frequency of each allele 50%) or greater than 2 allele polymorphism will be detected, if present, with a certainty of greater than 90% using such a screening panel.

Once polymorphism has been detected follow up gel transfer experiments can be done with panels of DNA (cleaved with the appropriate restriction enzyme) from more random unrelated individuals (e.g. 20) so that an estimate of the frequency of the RFLP alleles can be made and PIC can be estimated. In addition, other enzymes can be tested to see if additional variation can be detected. It is important to confirm that the polymorphism represents a single locus. This is done by studying the inheritance of the RFLP alleles in families. The presence of more than one locus would be suspected if non-Mendelian segregation of the banding pattern in the autoradiograms is observed. In some cases the polymorphic patterns are very complicated and alleles can only be defined from such inheritance studies. Useful family pedigrees for these analyses preferably contain the 4 grandparents, the parents, and large sibships (6 or more children) from which DNA can be purified from blood samples obtained or isolated from immortalized lymphoblastoid cell lines (Camden Cell Repository; White et. al., 1985).

Example 1:

In a first example of this invention the isolation and identification of Lam4-917 is described.

The isolation and characterization of Lam4-917 begins with the HaeIII-AluI partial digest library in the lambda vector Charon 4A (Lawn, et al. (1978) supra). The method used for isolating Lam4-917 is according to Wyman, et al (1980) supra and Maniatis, T., Hardison, R.C., Lacy, E., Laver, S., O'Connell, C., Quon, D, Sim, G.K., Efstradiatis, A (1978) Cell 15, 687-701.

Plaque hybridization of genomic DNAs is carried out by incubating approximately 150 bacteriophage of the HaeIII-AluI library in an equal volume of SM (50 mM Tris-HCl, pH 8.0, 100 mM NaCl, 8 mM $MgSO_4$) containing E. Coli strain CGE6 (CGE6 = HB101: F-, hsdS20($r-_B m-_B$), recA13, ara-14, proA2, lacYl, galK2, rpsL20 (Smr), xyl-5, mtl-1, supE44, lambda- as described by Bolivar, F. and Backman, K. (1979) Methods Enzymol. 68, 645, and Boyer, H. W. and Roulland-Dussoix, D. (1969) J. Mol. Biol. 41, 459) in a total volume of 2.5 ml. The mixture is incubated at 37°C for 15 minutes and then 3 ml LB top agarose is added, mixed and poured onto an LB (Luria Broth per liter: 10 g tryptone, 5 g yeast extract, 5 g NaCl, 15 g agar) plate. The plate is incubated at 37°C overnight. Following incubation, the plates are chilled at 4° C for one hour. Two plaque lifts per plate are done for one minute and two minutes. The plaque lifts on a filter are denatured for one minute, neutralized for five minutes, rinsed in 2X SSC (1X = 0.15 M NaCl, 0.015 M NaCitrate) and air dried for about two hours. The filters from both plaque lifts are baked at 80°C in a vacuum oven for two hours and then stored at room temperature.

Nick translation of the human probe is according to Rigby, et al. (1977) supra. Nicking the DNA is carried out for ten minutes at 13° C followed by five minutes at 65° C in a final volume of 8 µl containing DNase I cocktail (50 uUnits DNase I, 50 MM Tris, pH 7.5, 5 mM Mg $Cl_2$, 20 mM ß-mercaptoethanol, 0.05% gelatin), 0.25 µg human genomic DNA and 5 ng plasmid (pCGE40 contains about 10,000 copies of lac Z). The 8 µl nicked DNA (0.25 µg) was incubated at 13° C for 70 minutes with 7 µl Pol I cocktail (Pol I cocktail contains 50 µl $^{32}P$-dCTP from New England Nuclear, 10 µl 0.3 mM each dATP, dGTP and dTTP, 5 µl DNA polymerase I (50 units) from New England Biolabs and 5 µl distilled water for a final total volume of 70 µl. The reaction is terminated by the addition of 35 µl stop mix (stop mix contains 100 µl 0.5 M EDTA, 10 µl 10 mg/ml tRNA and 240 µl distilled water). The reaction mixture is loaded onto a 0.8 cm x 3.5 cm Biogel P-60 column preequilibrated with TE buffer (10 mM Tris, 10 mM $Na_3EDTA$ at pH 8.0 and washed with same. The location of the $^{32}P$-labeled human DNA probe is followed by counting samples of each fraction in a scintillation counter and the first peak is collected.

The labeled human DNA probe is hybridized to the blotted genomic DNA according to the following procedure. The first set of filters from the stored plaque lift filters are floated on 6X SSC and then submerged for about 10 minutes. The filters are prewashed in 660 ml prewash solution (50 mM Tris-HCl, pH 8.0, 1.0 M NaCl, 1.0 mM EDTA, 0.1% SDS) for about 2 hours in a 42° C shaker and then prehybridized in 150 ml prehybridization solution (5X SSC, 0.40 M sodium phosphate, pH 6.5, 0.1 mg/ml salmon sperm DNA, 10% dextran sulfate, 50% formamide, 5X Denhardt's Ficoll 400, polyvinylpyrrolidone 360, bovine serum albumin) for about 2.5 hours in a 42° C shaker. Following prehybridization, 200 µl of a solution containing greater than $10^8$ dpm human DNA probe is added and incubation in the 42° C shaker is continued overnight. Hybridization with the nick-translated probe is by the methods of Southern, E. M. (1975) J. Mol. Biol. 98, 503-517, and Barker, D. et al (1984) Am. J. Human Genetics 36 1159-1171 and are carried out for about 22 hours. Following hybridization, filters are washed for 30 minutes at room temperature in a solution containing 2X SSC and 0.1% sodium dodecylsulfate (SDS) and then 60 minutes at 65° C in 0.5X SSC and 0.5% SDS. Autoradiography was for 17 hours at -70° C with Kodak XAR-5 film, backed by a Lightening-Plus intensifying screen (DuPont).

The genomic DNA plaques undergo a second screening. This second screening is carried out as described above for plaque hybridization except that bacteria phage are applied to a lawn of strain CGE6 with a toothpick, nick translation of the human probe and hybridization of the labeled probe to the blotted DNA. Following two rounds of screening, Lam4-917 is found to hybridize to the genomic DNA, is a clear plaque and, therefore, a putative single copy DNA clone. A phage plate lysate is prepared as is well known in the art (Arber, W., Enquist, L., Hohn, B., Murray, N. and Murray, K. (1983) Exp. Methods for Use with Lambda, Lambda II, ed. P. Hendricks, J. Roberts, F. Stahl, R. Weisberg, 433-466 Cold Spring Harbor) from the clear plaque identified as Lam4-917. To obtain the phage lysate, approximately $10^5$ phage particles are incubated with 100 µl CGE6 overnight at 37° C for 15 minutes. LB top agarose (3 ml) is added to the incubated solution and then the total solution is poured onto LB plates. The plate is incubated at 37° C for 8 hours and then 3.5 SM is added to the plate and the plate is incubated at 4° C overnight. The phage titer is about 1-2 X $10^{10}$ pfu/ml. The SM is pipetted from the phage lysate, a few drops of chloroform is added and the solution is vortexed for about 10 seconds. The lysate is spun in two microcentrifuge tubes for 15 minutes at 4° C, and then transferred to a screw-cap tube and stored at 4° C.

A DNA miniprep is prepared from the phage according to Davis, R. W., Botstein, D. and Roth, J. R. (1980) " A Manual for Genetic Engineering. Advanced Bacterial Genetics", Cold Spring Harbor Laboratory, Cold Spring Harbor, N. Y., 109-111 and left in isopropanol at -20° C overnight. The phage DNA is collected following centrifugaton in a microcentrifuge for 15 minutes at 4° C. The DNA pellet is air-dried and resuspended in 20 µl TE, pH8.0. A 100 ng sample of the phage DNA (1 µl DNA plus 9 µl stop mix) is run on a 1% agarose gel in TA buffer at 60 volts (about 90 mAmps) together with a lambda DNA control to determine that the human DNA clone is isolated and results confirm that the clone of interest is isolated.

Lam4-917 is nick translated using conditions described previously and hybridized to restriction enzyme cleaved genomic DNA. The restriction enzyme cleaved genomic DNA blot consists of five unrelated individual DNA restriction cleaved with each of six restriction enzymes: MspI, TaqI, HindIII, EcoRI, BglII and BamHI. The genomic DNA are restricted separately with the appropriate restriction enzyme according to the following procedure. Enough of the DNA solution for 100µg (usually 400ul) is added to a 1.5 ml Eppendorf tube together with the appropriate amount of the specific endonuclease digestion buffer made to the manufacturer's recommendations, restriction endonuclease in a 2 to 5 fold excess, 2 mM spermidine-HCl and distilled water, if necessary, to adjust to the final reaction volume of 600 µl. The mixtures are incubated at 37° C overnight with the exception of the TaqI digests which are incubated at 65° C. The human DNA is monitored for complete digestion by digesting lambda phage DNA in the presence of a sample of the human digest. Digestion of the human DNA is judged to be complete if the lambda DNA is fully digested. The reaction is terminated with stop reactants (final concentration - 10 mM EDTA, pH 7.0, 0.5% SDS and 0.3 M NaCl) and stored for future electrophoresis. Prior to electrophoresis, marker dye is added to each sample and then loaded on the gel. The DNAs are subjected to electrophoresis on 0.8% agarose gels submerged in a Tris-acetate ADTA buffer. Following electrophoresis the DNA is denatured in th gel with a solution of 0.2 N NaOH, 0.6 M NaCl, neutralized with 0.5 M Tris-HCl pH 8.0, 1.5 M NaCl and transferred overnight (16 hours) to a nylon membrane (zeta-bind, AMF Cuno) in 25 mM sodium phosphate pH 6.5. The nylon membrane is washed then baked for 2 hours in a vacuum oven at 80° C. The DNA containing nylon membrane is then prehybridized 2 hours at 42° C in a solution containing 5x SSC 40 mM sodium phosphate, pH 6.5, 0.1 mg/ml salmon sperm DNA, 10% dextran sulfate, 50% formamide, 5x Denhardts Ficoll 400, polyvinylpyrrolidone 360, bovine serum albumin. The RFLP 917 DNA probe is labeled with $^{32}P$ to a specific activity of greater than $10^8$dpm/g via a nick translation reaction with DNA polymerase I (New England Biolabs) and alpha-$^{32}P$ NTPs (New England Nuclear) as described previously. Labeled DNA is separated from unincorporated nucleotides by chromatography on Biogel P-60 (Bio-Rad). The $^{32}P$ labeled DNA is denatured by boiling for 5 minutes then added to the prehybridization solution containing the nylon membrane. Hybridization is carried out at 42° C for 20 hrs. Following hybridization, filters are washed as described previously. Autoradiography is done for 1-5 days at -70° with Kodak XAR-5 film and intensifying screens (Dupont Cronex Lightening Plus).

The results of the Southern hybridization show that Lam4-917 can detect a HindIII two allele polymorphism. The HindIII polymorphism is verified by rescreening Lam4-917 on a HindIII panel of human genomic DNA as described previously. Results are consistent with the previous finding that Lam4-917 reveals a HindIII two allele polymorphism. In addition, a HincII polymorphism is revealed on the former panel which revealed the HindIII polymorphism. The Lam4-917 marker is rescreened on a HincII genomic DNA panel and Southern hybridization results verify that Lam4-917 can detect both a HindIII two allele polymorphism and a HincII polymorphism.

The restriction map of Lam4-917 is determined by the cos-oligonucleotide method according to Rackwitz, H., Zehetner, G., Frischauf, A. and Lehrach, H. (1984) Gene 30, 195-200 and by standard restriction analysis of subcloned EcoRI fragments (Table 2). The correspondence of the restriction sites in the clone, which contains 18 Kb (kilobases) of human DNA, with those in the human genome is confirmed by Southern gel-transfer hybridization analysis as described previously.

Linkage of Lam4-917 to the major CF locus is made by application (hybridization) of the 917 RFLP probe to 39 nuclear families containing two or more offspring with positively diagnosed CF. The data is analyzed by the likelihood method of linkage analysis via a standard computer program (LIPED 3.3) available to one skilled in the art of human genetics and according to Ott, J. (1974) Amer. J. Human Gen. 26, 588-597 and Ott, J. (1985) "Analysis of Human Genetic Linkage", in The Johns Hopkins Univ. Press, Baltimore, Md., 22-119 (Table 3). The LOD score is a measure of the relative likelihood that there is linkage (at a given recombination fraction) and the alternative hypothesis that the date arose by chance and no linkage exists. The value generally accepted as conclusive is a LOD score of 3. The LOD score for Lam4-917 is 3.96 at a recombination fraction of 0.14. Formally, such a score means that the odds are about 10,000 to 1 that there is linkage at this distance as opposed to non-linkage. In order to determine if there is linkage between CF and PON, paraoxonase typing data is analyzed from the CF families. Eleven of the 31 families with available data are informative for this analysis. A tight linkage is detected between Lam4-917 and PON (Table 4). A maximal likelihood estimate for O at 0.05 is obtained with a LOD score of 5.01 (odds ratio: 102,000:1) and the confidence interval between 0.01 and 0.17. A maximal LOD score of 5.13 (odds ratio: 132,000:1) is obtained at $O_M$ of 0.10 and $O_F$ of 0.04.

The probe Lam4-917 was hybridized to DNA from a set of rodent-human hybrid cell lines containing various subsets of the human chromosome complement (Showo, T.B., Sakaguchi, A.Y., Naylor, S.L. (1982) Adv. Hum. Genet. 12, 341-452). Results from 25 cell lines tested indicate that the clone Lam4-917 is derived from human chromosome 7. Hybridization results are concordant with the presence or absence of chromosome 7 in 22 lines, and discordant in only one.

Assignment of Lam4-917 to chromosome 7 was testing additional somatic cell hybrid panels lines and by *in situ* hybridization to metaphase chromosomes.

Example 2:

This example illustrates diagnosis of cystic fibrosis in counseling situation.

A typical counseling situation appropriate for application of the CF RFLPs test of this invention is one shown by the family pedigree in Table 4; a CF child is born thus indicating to the parents that they each carry a CF allele. The parents wish to continue having children and want to know the status of a living fetus at risk carried by the mother, i.e. is it affected with cystic fibrosis, is it a carrier or is it free of either disease gene.

The CF allele at the locus revealed by the Lam4-917 probe is responsible for CF in this family and accordingly the alleles revealed by Lam4-917 can be used to follow CF in this family.

Blood samples (1-20 ml) are collected from the mother, father and affected child and the DNA is purified according to the following procedure. Cells are first lysed by addition of a ten fold volume of buffer (0.32 M sucrose, 10 mM Tris-HCl pH 7.6, 5 mM $MgCl_2$, 1% triton - X-100) and nuclei are isolated by centrifugation. The nuclear pellet is then suspended in a small volume of 75 mM NaCl, 25 mM EDTA, 1% SDS and incubated with proteinase K (10mg/ml) overnight (12-16 hrs.) at 42°C. Following a 2-4 hour extraction at room temperature of the solution with Tris-EDTA saturated phenol, the aqueous solution is re-extracted with a solution of chloroform and isoamylalcohol (24:1) and the DNA is precipitated by addition of one tenth volume of 3 M NaCl and 1.5 volume isopropanol. The DNA is dissolved in 0.1 x TE buffer and reprecipitated by addition of 1/2 volume 7.5 M ammonium acetate and 2 volumes of cold 95% ethanol. The precipitated DNA is resuspended in 10 mM Tris-HCl, pH 7.5, 1mM EDTA to a concentration of 250 ug/ml and stored at 4° C. The DNA yield is approximately 100-200 ug per 10 ml peripheral blood of 10 ug per $10^6$ cells.

DNA is obtained from the fetus at risk as from the cells obtained from an amniotic fluid sample. The DNA is purified as described above.

The four DNAs (father, mother, affected child and fetus at risk) are each digested with restriction enzymes HindIII and HincII separately. DNA samples at a final concentration of 175 µg/ml are digested to completion (4-6 hours) with restriction endonucleases HindIII and HincII (5 units restriction enzyme/µg DNA) under conditions recommended by the supplier (New England Biolabs). In order to check the completeness of the digestion aliquots from the reaction mixture containing 1 µg human genomic DNA are added to an identical reaction mixture containing 1 ug bacteriophage lambda DNA. A limit digest of the genomic DNA is assumed if the digest pattern of lambda DNA showed complete cleavage as measured by ethidium bromide staining of the DNA size-fractionated by electrophoresis in a 0.8% agarose gel (Sigma). Fragments from the four restricted DNAs are separated according to size by electrophoresis through a 0.8% agarose gel and transferred to a solid support nylon membrane as described in Example 1. DNA probe Lam4-917 is radiolabeled with $^{32}P$ and hybridized to the enzyme cleaved DNAs from the 4 family members. Results are interpreted from autoradiographs of the hybridized membrane.

As shown in Table 5, since the affected child carries both mutant alleles at the CF locus, the RFLP alleles revealed at the 917 locus are those associated with the mutant CF allele itself thus alleles B and D (HindIII digest) and F and H (HincII pattern) correspond to the mutant alleles. Therefore these alleles are the mutant alleles carried by each parent whereas the alleles ACEG are linked to normal CF genes in the parents. Analysis of the hybridization pattern of the fetus at risk shows that alleles ACEG are inherited from the parents indicating that the child has a normal genotype and will not develop CF or pass on a mutant gene to any offspring except that a 15% recombination factor has to be considered for each allele of 917 because of the genetic distance between the CF locus and the 917 locus. Therefore the diagnosis must be interpreted with this probability factor included.

Example 3:

This example illustrates diagnosis of cystic fibrosis using flanking markers.

A CF child is born thus indicating to the parents that they each carry a CF allele. The parents wish to continue having children and want to know the status of a living fetus at risk carried by the mother, i.e., is it affected with cystic fibrosis, is it a carrier or is it free of the disease gene.

The CF allele at the locus is revealed by two flanking markers (Lam4-917 and X where X is S-094, S-002, L4-1033 or S-158). For example illustration, the markers are Lam4-917 and S-002 and they are fully informative in this family and accordingly the alleles revealed by them can be used to follow CF in this family. DNA is obtained, digested with restriction enzymes (HindIII and IncII for Lam4-917 and TaqI for S-002) and probed with the markers as described in Example 2.

Analysis of the hybridization patterns of the fetus at risk shows inherited alleles from the parents are normal alleles indicating that the child has a normal genotype and will not develop CF or pass on a mutant gene to any subsequent offspring. Since in Example 2 only one marker was being used, a 15% recombination factor had to be considered for each allele of Lam4-917 because of the genetic distance between the CF locus and the locus as defined by Lam4-917 and the probability that the diagnosis is correct using this one marker is about 70%. In the present Example using flanking markers and where no crossover has occurred, there is a 15% recombination factor between S-002 and Lam4-917 and a 5% recombination factor between S-002 and CF making the percent error of 0.4% or a probability of accuracy of 99.6% in this case.

While specific embodiments of the invention have been shown and described above, many variations are possible. The DNA used for testing can be obtained from blood samples, amniotic fluid, chorion tissue samples or from other biofluids and cells where DNA is present.

The DNA can be purified from the samples taken in any known manner. Normally the cells are treated to rupture the cell walls and release the contents whereupon foreign matter and proteins are removed from the DNA so as to purify it before testing. Conventional centrifugation, extractions and the like can be used.

The restriction digesting procedure can use standard techniques known in the art. While HindIII and HincII restriction enzymes are used with Lam4-917, other restriction enzymes can be used when other CF locus identifying probes are used as known in the art.

The electrophoresis step can use parameters known in the art for separating restriction digested DNA segments.

BAD ORIGINAL

The solid support to which the electrophoretically separated DNA is transferred can be a nylon membrane, paper or other solid supports as known in the art. In some cases, the support need not be used if good separation of digested DNA segments can be obtained in a readable form using standard techniques.

Hybridization techniques known in the art can vary greatly. The double stranded DNA to be tested is disassociated as with suitable chemicals, temperature or varied ion concentrations. The single copy probe is then applied to single stranded DNA, under conditions which permit rehybridization.

In some cases the use of restriction enzymes can be carried out simultaneously with deposit and/or separation of the DNA in a suitable support.

While the invention specifically describes use of a single probe in each test as in Example 2, plural probes can be used in each test in the testing procedure as in Example 3. Such multiple probes can be flanking markers or multiple linked probes.

We hereby incorporate by reference all of the publications recited herein and also the attached paper entitled "Cystic Fibrosis Locus Defined by a Genetically Linked Polymorphic DNA Marker" by Lap-Chee Tsui et al pages 0-21.

BAD ORIGINAL

CLAIMS

1. A method of determining presence or absence of one or more cystic fibrosis alleles in a DNA sample taken from an individual having a family genetic background in which family, DNA of ancestors in the family line contains identified cystic fibrosis causing allele genes, said method comprising,

restriction digesting a DNA sample to be tested, which DNA sample is from an individual having a family genetic background in which family ancestors of the family have DNA which contains identified cystic fibrosis allele;

applying a probe to said DNA sample which probe hybridizes within less than 50 centimorgans of a cystic fibrosis gene in said family DNA,

and determining whether or not hybridization has taken place in a manner consistent with the presence of one or more cystic fibrosis alleles in said sample.

2. A method in accordance with the method of claim 1 wherein said DNA is first purified by rupturing cell walls and obtaining said DNA sample to be tested in purified form prior to restriction digesting.

3. A method in accordance with claim 2 and further comprising separating said restriction digested DNA by electrophoresis prior to applying said probe.

4. A method in accordance with the method of claim 3 and further comprising transferring said electrophoretically separated DNA to a solid support prior to applying said probe.

5. A method in accordance with the method of claim 1 wherein said probe hybridizes within 15 centimorgans of said cystic fibrosis gene and said digesting is carried out with Hind III and Hinc II.

6. A method in accordance with the method of claim 5 wherein said probe carries a marker for identifying DNA band positions which exhibit hybridization on said solid support.

7. A method in accordance with the method of claim 4 wherein said determining is carried out by comparing the results of hybridization with the results obtained from DNA of the parents and a sibling exhibiting the disease of the individual from whom the sample to be tested was taken, so that hybridization at similar positions on the support with respect to each parent and a sibling exhibiting the disease indicates the presence or absence of a cystic fibrosis allele obtained from each parent in the DNA tested.

8. A method in accordance with the method of claim 5 wherein said determining is carried out by comparing the results of hybridization with the results obtained from DNA of the parents and the sibling exhibiting the disease of the individual from whom the sample to be tested was taken, so that hybridization at similar positions on the support with respect to each parent and sibling indicates the presence or absence of a cystic fibrosis allele obtained from each parent in the DNA tested.

9. A method in accordance with claim 1 wherein said probe is Lam4-917.

10. A method in accordance with claim 7 wherein said probe is Lam4-917.

11. A method in accordance with claim 4 wherein said probe is Lam4-917.

12. In a method of identifying whether or not an allele of a cystic fibrosis gene is present in a DNA sample taken from an individual, the improvement comprising applying a probe under hybridizing conditions to said DNA sample which probe identifies a cystic fibrosis locus specific to a cystic fibrosis gene in said DNA if present and determining whether or not said probe has hybridized to said DNA.

13. A method in accordance with the method of claim 12 wherein said probe hybridizes within less than 50 centimorgans of a cystic fibrosis gene locus.

14. A method in accordance with the method of claim 12 wherein said probe hybridizes within 15 centimorgans of a cystic fibrosis gene locus.

15. A method in accordance with the method of claim 12 wherein said probe has a PIC value of at least 0.58.

16. A method in accordance with the method of claim 12 wherein said probe is Lam4-917.

BAD ORIGINAL

17. A probe which hybridizes within less than 50 centimorgans of a cystic fibrosis gene locus.

18. A probe which hybridizes within 15 centimorgans of a cystic fibrosis gene locus.

19. Lam4-917.

20. In a method of determining the presence or absence of a cystic fibrosis gene, the use of a DNA probe obtained from a gene library of human genomic DNA, said probe having a PIC value of at least 0.58.

21. A method in accordance with the method of claim 20 wherein said probe is applied to DNA to be tested after digesting said DNA to be tested with Hind III and Hinc II.

22. A method of determining presence or absence of one or more autosomal recessive trait alleles in a DNA sample taken from an individual having a family genetic background in which family, DNA of ancestors in the family line contains said trait, said method comprising, obtaining a DNA sample from said individual,

restriction digesting said DNA sample to be tested,

applying a probe to said DNA sample which hybridizes within less than 50 centimorgans of a gene for said autosomal recessive trait, in said family DNA,

and determining whether or not hybridization has taken place in a manner consistent with the presence of one or more said alleles in said sample.

23. In a method of identifying whether or not an autosomal recessive trait gene is present in a DNA sample taken from an individual, the improvement comprising applying a probe under hybridizing conditions to said DNA sample which probe identifies an autosomal recessive trait locus specific to an autosomal recessive trait gene in said DNA if present and determining whether or not said probe has hybridized to said DNA.

24. A cystic fibrosis gene locus which is within 15 centimorgans of a portion of attached DNA which portion hybridized to Lam4-917.

25. A method in accordance with the method of Claim 1 and further comprising applying at least one additional probe to said restriction digested DNA sample to be tested.

26. A method in accordance with the method of Claim 25 wherein said at least one additional probe is a flanking marker to said first mentioned probe.

27. The improvement of Claim 20 wherein a second DNA probe obtained from a gene library of human genomic DNA having a pick value of at least 0.58 is used in conjunction with said first mentioned probe and is a flanking marker to said first mentioned probe.

29. A method in accordance with the method of Claim 22 and further comprising applying a second probe to said DNA sample after said restriction digesting, which second probe hybridizes within less than 50 centimorgans of a gene for said autosomal recessive trait in said family DNA and which second probe is a flanking marker to said first mentioned probe.

30. A cystic fibrosis gene locus which is within 15 centimorgans of a portion of attached DNA which acts as a marker hybridized thereto on one side of said locus and a second portion of attached DNA which second portion hybridizes on a second side of said gene locus.

Table I. -

Probe - Lam4-917

Enz - HindIII

PIC - 0.36

# Chromosomes examined - 32

Alleles/ Fragment lengths - (1) 6.2 Kb
(2) 5.1, 1.0

Allele Frequency - (1) 0.625
(2) 0.375

Const. Fragments (Kb) - 12.5, 7.6

related Pm - none

Unrelated Pm - HincII

---

Probe - Lam4-917

Enz - HincII

PIC - 0.35

# chromosomes examined - 32

Alleles/ Frag lengths (Kb) - (1) 4.3
(2) 2.3, 1.8

Allele Frequencies - (1) 0.67
(2) 0.34

Const frags (Kb) - 6.8, 5.1, 3.9

Related Pm - none

Unrelated Pm - HindIII

---

Combined PIC HindIII + HincII = 0.58

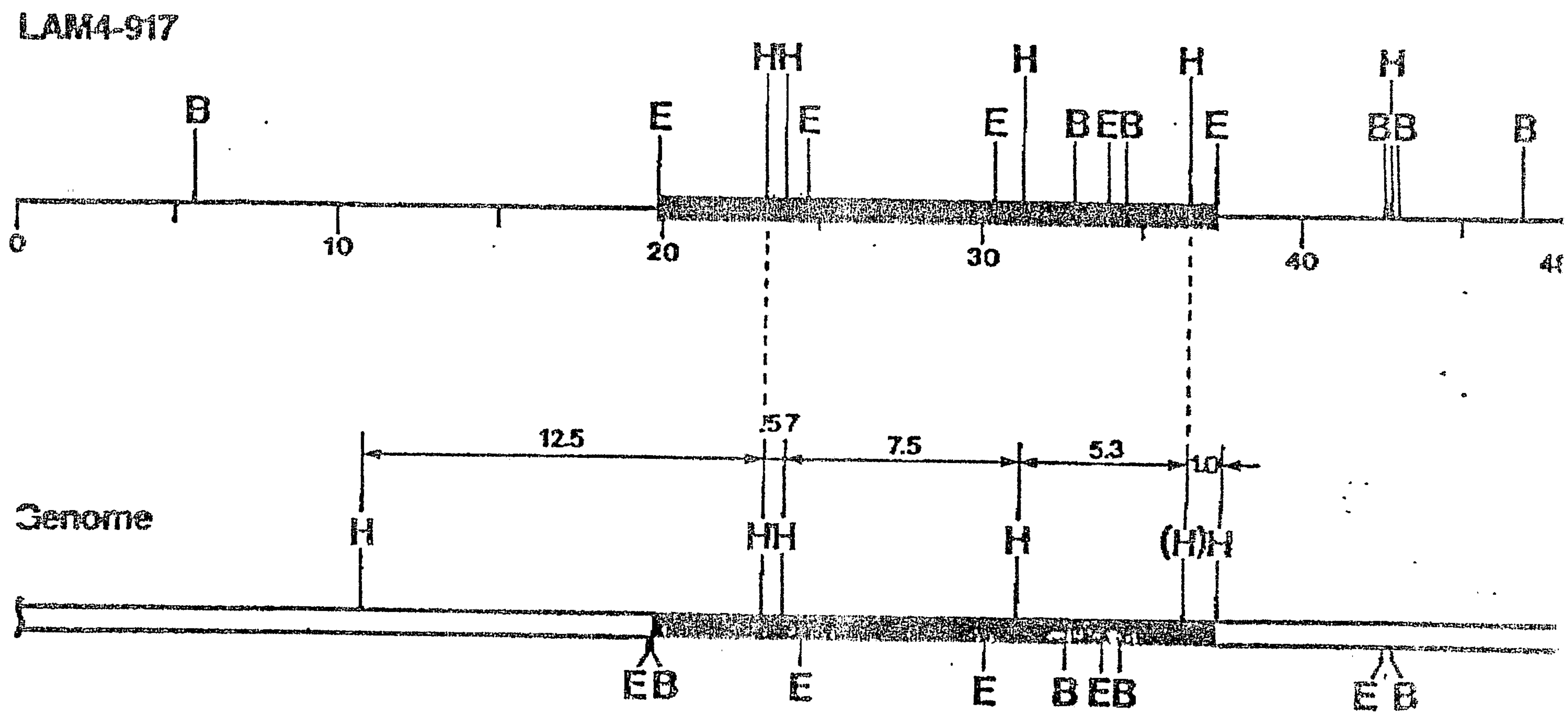
LAM4-917
B
E
HH
E
E
H
BEB
H
E
H
BB
B
0
10
20
30
40
Genome
12.5
.57
7.5
5.3
1.0
H
HH
H
(H)H
EB
E
E
BEB
E B

TABLE 2

TABLE 3

# Linkage Relationships

| Loci | Number of informative families | LOD (z) scores at recombinant fractions ($\theta$) of: | | | | | | | | |
|---|---|---|---|---|---|---|---|---|---|---|
| | | 0.01 | 0.05 | 0.10 | 0.15 | 0.20 | 0.25 | 0.30 | 0.35 | 0.40 |
| Lam4-917–CF | 36 (Can) | –6.02 | 0.98 | 2.84 | 3.20 | 2.96 | 2.44 | 1.79 | 1.13 | 0.55 |
| | 3 (HGCMR) | 0.14 | 0.69 | 0.79 | 0.75 | 0.66 | 0.53 | 0.39 | 0.25 | 0.12 |
| 39 | Total | –5.88 | 1.67 | 3.63 | 3.95 | 3.62 | 2.97 | 2.19 | 1.38 | 0.67 |
| | | [$\hat{\theta}$ = 0.14 (z = 3.96); confidence interval: 0.07–0.25; $\hat{z}$ = 4.13 ($\theta_M$ = 0.09; $\theta_F$ = 0.19)] | | | | | | | | |
| Lam4-917–PON | 11 (Can) | 4.27 | 5.01 | 4.78 | 4.28 | 3.56 | 2.97 | 2.25 | 1.51 | 0.81 |
| | | [$\hat{\theta}$ = 0.05 (z = 5.01); confidence interval: 0.01–0.17; $\hat{z}$ = 5.12 ($\theta_M$ = 0.10; $\theta_F$ = 0.04)] | | | | | | | | |

Mode of Inheritance Recessive

Risk Summary:

Fully Informative RFLP Marker, θ = 0.15

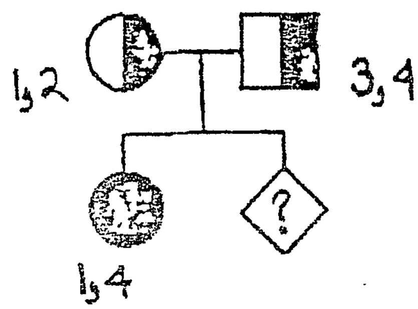


TABLE 4

TABLE 5

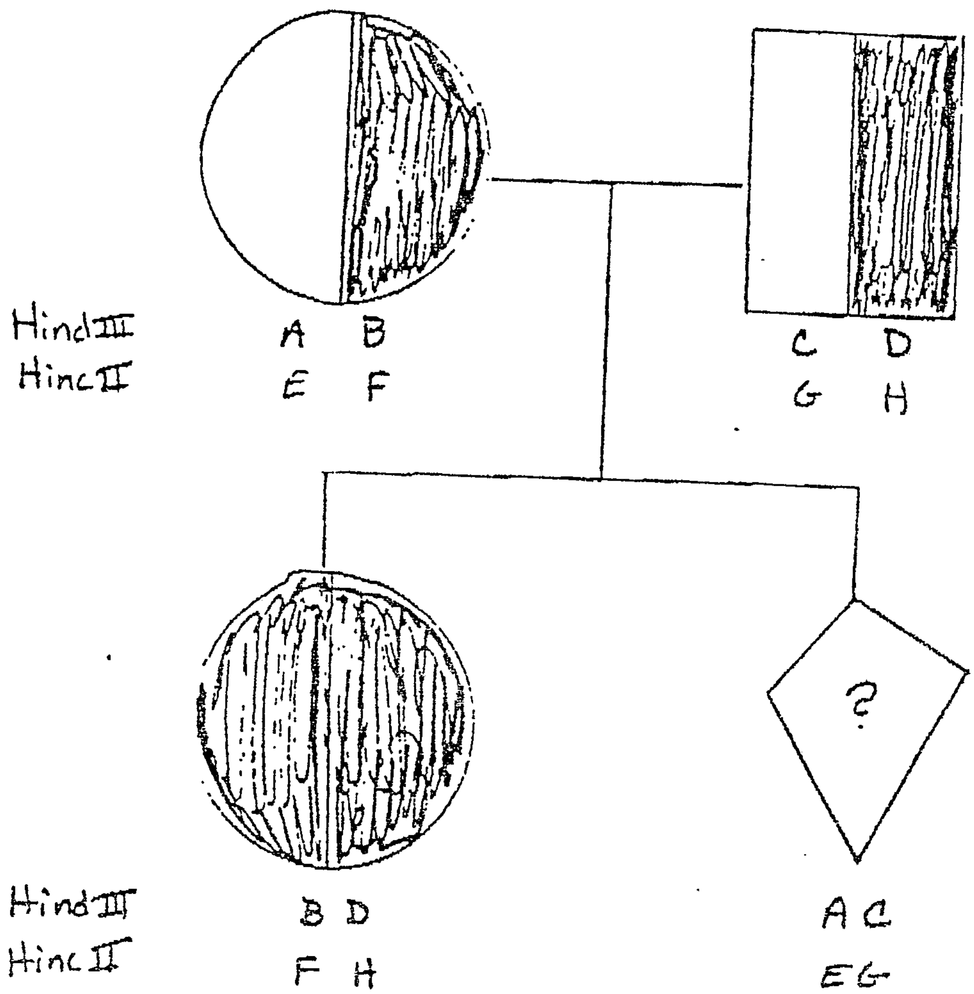

# TABLE 6

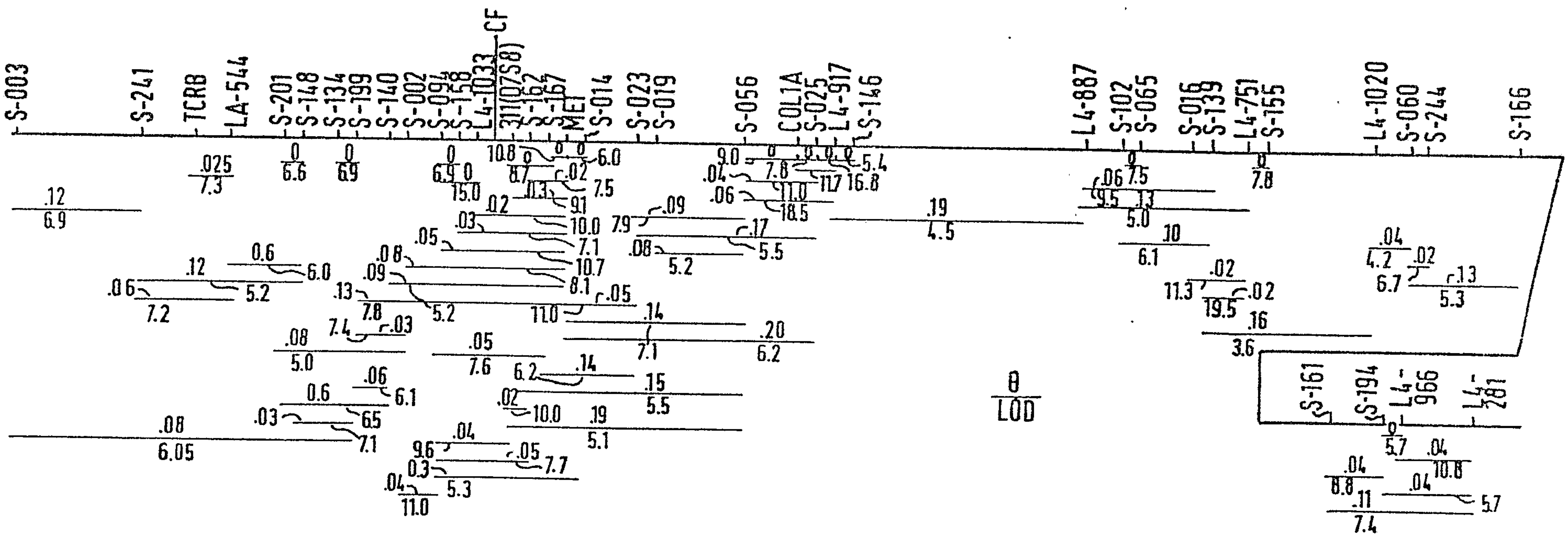
S-003
S-241
TCRB
LA-544
S-201
S-148
S-134
S-199
S-140
S-002
S-094
S-158
L4-1033
CF
311(0758)
S-162
S-167
MET
S-014
S-023
S-019
S-056
COL1A
S-025
L4-917
S-146
L4-887
S-102
S-065
S-016
S-139
L4-751
S-155
L4-1020
S-060
S-244
S-166
S-161
S-194
L4-966
L4-281
θ
LOD

Neu eingereicht / Newly filed
Nouvellement déposé

Table 7



| Probe | Enzyme | Variable Fragment Length (Kb) | Constant Fragment Length (Kb) |
|---|---|---|---|
| S-140 | Msp I | Allele 1 - 5.4 Kb | 1.7 Kb |
| | | Allele 2 - 5.7 Kb | |
| S-002 | Msp I | Allele 1 - 4.3 Kb, 2.4 Kb | 1.0 Kb |
| | | Allele 2 - 2.9 Kb, 2.0 Kb | |
| S-094 | Taq I | Allele 1 -19.0 Kb | none |
| | | Allele 2 -14.0 Kb, 7.8 Kb | |
| S-158 | Taq I | Allele 1 - 6.5 Kb | 3.3 Kb, 2.2 Kb |
| | | Allele 2 - 5.3 Kb | |
| L4-1033 | Msp I | Allele 1 -12.4 Kb | 8.8 Kb |
| | | Allele 2 -10.5 Kb | |
| S-162 | Msp I | Allele 1- 10.9 Kb | 2.3 Kb |
| | | Allele 2 -10.4 Kb | |
| S-157 | Msp I | Allele 1- 9.8 Kb, 3.8 Kb | none |
| | | Allele 2 - 2.7 Kb, 1.7 Kb, 1.2 Kb | |
| S-014 | Msp I | Allele 1 -18.0 Kb | 8.0 Kb |
| | | Allele 2 -15.0 Kb | |
| | | Allele 3 -14.5 Kb | |
| S-023 | Taq I | Allele 1 -10.5 Kb | 1.0 Kb |
| | | Allele 2 - 3.4 Kb | |